(19) Europäisches Patentamt
European Patent Office
Office européen des brevets

(11) **EP 2 142 202 B1**

(12) **FASCICULE DE BREVET EUROPEEN**

(45) Date de publication et mention
de la délivrance du brevet:
**15.01.2014 Bulletin 2014/03**

(51) Int Cl.:
***A61K 36/185*** *(2006.01)*   ***A61K 8/97*** *(2006.01)*
***A61P 17/14*** *(2006.01)*   ***A61Q 7/00*** *(2006.01)*

(21) Numéro de dépôt: **08735813.1**

(86) Numéro de dépôt international:
**PCT/EP2008/054072**

(22) Date de dépôt: **04.04.2008**

(87) Numéro de publication internationale:
**WO 2008/125520 (23.10.2008 Gazette 2008/43)**

(54) **EXTRAIT DE CLEOME SPINOSA POUR SON UTILISATION DANS DES PREPARATIONS PHARMACEUTIQUES ET COMPOSITIONS COSMETIQUES**

EXTRAKT VON CLEOME SPINOSA IN PHARMAZEUTISCHEN ZUBEREITUNGEN UND KOSMETISCHEN ZUSAMMENSETZUNGEN

CLEOME SPINOSA EXTRACT USED IN PHARMACEUTICAL PREPARATIONS AND COSMETIC COMPOSITIONS

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **05.04.2007   FR 0754329**

(43) Date de publication de la demande:
**13.01.2010   Bulletin 2010/02**

(73) Titulaire: **Pierre Fabre Dermo-Cosmétique**
**92100 Boulogne-Billancourt (FR)**

(72) Inventeurs:
• **BELLE, René**
  **F-81710 Saix (FR)**
• **BELAUBRE, Françoise**
  **31270 Villeneuve Tolosane (FR)**

(74) Mandataire: **Regimbeau**
**20, rue de Chazelles**
**75847 Paris Cedex 17 (FR)**

(56) Documents cités:
• **DATABASE WPI Week 200166 Thomson Scientific, London, GB; AN 2001-586328 XP002456355 & JP 2001 181129 A (SHISEIDO CO LTD) 3 juillet 2001 (2001-07-03)**
• **DE ALBUQUERQUE ULYSSES PAULINO: "Re-examining hypotheses concerning the use and knowledge of medicinal plants: a study in the Caatinga vegetation of NE Brazil" JOURNAL OF ETHNOBIOLOGY AND ETHNOMEDICINE, BIOMED CENTRAL, LONDON, GB, vol. 2, no. 1, 26 juillet 2006 (2006-07-26), page 30, XP021020084 ISSN: 1746-4269**

**Description**

La présente invention concerne le domaine général des préparations pharmaceutiques ou cosmétiques traitant la chute des cheveux.

*Cleome spinosa* Jacq. qui porte comme synonyme *Cleome hassleriana* Chodi est une herbacée de la famille des Capparidaceae et est originaire du Sud Est du Brésil et d'Argentine. Elle est très largement cultivée comme plante ornementale et de nombreux cultivars agrémentent terre-pleins et ronds-points de nos villes durant l'été de fin juin à septembre.

Elle est parfois appelée communément « fleur araignée ».

Le nom du genre *CLEOME* aurait pour origine étymologique le mot grec Kleos qui signifie « Gloire ».

Le genre *Cleome* comprend des herbacées annuelles, des sous arbustes, rarement des lianes. Ils peuvent être inermes ou armés de stipules épineuses, les tiges et les feuilles sont souvent pubescentes avec des poils glandulaires, à odeur forte.

Les feuilles sont alternes, digitées de 3 à 9 folioles sur un pétiole allongé, ou unifoliolées et alors sessiles.

Les fleurs, bisexuées, sont groupées en racèmes ou solitaires axillaires. Elles sont généralement zygomorphes et composées de 4 sépales libres, 4 pétales spatulés, à disque nectarifère. Les étamines au nombre de 6 sont exsertes.

Le fruit cylindrique est une silique déhiscente par 2 valves s'écartant du replum (ou fausse cloison) pour libérer des graines nombreuses, réniformes plus ou moins sphériques.

Le genre comprend environ 80 espèces néotropicales dont 19 au Venezuela.

L'espèce *Cleome spinosa* Jacq. se différencie des autres espèces par les dimensions plus importantes de ses organes fructifères : gynophore de 20 jusqu'à 50 mm (contre 2 à 9 mm), fleurs grandes à pétales de 10 à 25 mm de long, blancs ou teintés de pourpre, étamines pourpres. Les feuilles comportent 5 à 7 folioles.

*Cleome spinosa* Jacq. est une plante annuelle dressée de 1 à 2 m de haut, glanduleuse, visqueuse, très épineuse. Fleurs s'épanouissant en été, à pétales plus courts que les étamines. Gynophore séparant les étamines du disque. Fruit linéaire de 6 à 12 cm de long, dressé.

L'huile des graines, riche en glucosinolates est utilisée en médecine empirique contre les maux d'oreilles et autres maladies.

Les parties aériennes ne font pas l'objet d'utilisation en médecine traditionnelle. Par contre la plante est largement utilisée comme plante ornementale.

*Cleome spinosa* a fait l'objet de très peu d'étude scientifique aussi bien sur le plan chimique que pharmacologique. Une activité du *Cleome spinosa* comme inhibiteur de collagénase a été décrite ainsi que son utilisation comme « anti-âge » (JP2001181129).

Dwight O. *and al.* ont travaillé sur le plan phytochimique sur les parties aériennes de *Cleome spinosa* (Dwight and al.l, J. Nat. Prod. 2004, 67, 179-183). Ils décrivent ainsi en plus d'une flavone, la flindulatine, cinq nouveaux cembranes appartenant à la famille des diterpènes, appelés cleospinol A, B, C, D et l'ester 3'-hydroxy-iso-pentane-10-oate de cleospinol A.

Les graines avaient fait déjà l'objet d'une étude décrivant la présence de glucosinolates, en particulier la glucocapparine [Pagani K. and al. coll, Il Farmaco, Ed. Prat. 22, 553 (1967)].

De façon inattendue et surprenante, la demanderesse a mis en évidence l'utilisation d'un extrait de *Cleome spinosa* en tant que principe actif pour la préparation d'une composition pharmaceutique ou cosmétique destinée à prolonger la croissance de follicules pileux.

La présente invention est définie dans les revendications.

La présente invention porte sur un extrait de *Cleome spinosa* en tant que principe actif d'une composition pharmaceutique pour son utilisation pour le traitement des désordres capillaires, ainsi que sur un procédé cosmétique pour prolonger la croissance de follicules pileux, caractérisé en ce qu'il implique l'utilisation par voie topique ou orale d'un extrait de *Cleome spinosa.*

L'extrait de *Cleome spinosa* qui présente une teneur globale en soufre comprise entre 0,05 g et 5g pour 100 g de matière sèche de l'extrait est décrit.

Ledit extrait de *Cleome spinosa* peut contenir des acides aminés soufrés, en particulier la cystine et la méthionine.

Ledit extrait peut contenir de 0,01 g à 1g de cystine pour 100 g de matière sèche de l'extrait.

Ledit extrait peut contenir de 0,001 g à 0,1 g de méthionine pour 100 g de matière sèche de l'extrait.

Des compositions cosmétiques topiques ou orales contenant un tel extrait de *Cleome spinosa* sont également décrites.

Le cheveu comprend: la tige pilaire (composée essentiellement d'une protéine soufrée "la kératine" et de pigments "la mélanine" qui déterminent sa couleur); et le bulbe pileux (racine même du cheveu), entouré de la zone "péri-bulbaire" où se trouvent en particulier les vaisseaux sanguins qui transmettent tous les éléments nutritifs essentiels.

Les cheveux ne poussent pas continuellement mais selon un cycle de croissance pilaire divisé en 3 phases.

Les phases de croissance (phase anagène), de régression (phase catagène) et de repos (phase télogène) se succèdent. Pendant cette dernière phase, le cheveu est progressivement délogé par un nouveau cheveu anagène issu du même follicule. Le cheveu natif va provoquer la chute du cheveu mature.

La chute de cheveux - diffuse ou localisée - , en tant que désordre capillaire, se caractérise par l'augmentation du nombre de cheveux en phase « catagène ».

La demanderesse a montré qu'un extrait de Cleome spinosa stimule l'élongation de la tige pilaire.

Dans le cadre de la présente invention, l'extrait de Cleome spinosa maintient, prolonge la phase anagène du cycle précédemment décrit. Au cours de cette phase de croissance, le cheveu se forme et croît par une division des cellules du follicule pileux. Le follicule est alors implanté dans le tissu hypodermique.

La phase anagène est primordiale pour l'élongation de la tige pilaire du cheveu.

La composition selon la présente invention destinée à stimuler et à prolonger la croissance de follicules pileux comprend un extrait de *Cleome spinosa* comme principe actif.

Dans le cadre de la présente invention, on entend par désordres capillaires toute modification touchant à la structure ou au fonctionnement du follicule pileux. Plus particulièrement, par désordres capillaires, on entend toute perturbation biologique entraînant la perte occasionnelle ou prolongée des cheveux. Les désordres capillaires peuvent être d'origine occasionnelle ou installée.

Les désordres capillaires peuvent avoir différentes causes d'origine interne ou externe. Par origine interne, on entend toute perturbation touchant les processus biologiques impliqués dans la croissance et le maintien du cheveu. On citera par exemple le dérèglement sébacé, l'amplification enzymatique des 5-alpha-réductases (dérèglement hormonal), l'inflammation, les modifications structurales du collagène, l'intervention des facteurs de croissance, des facteurs nerveux ou des facteurs vasculaires et la composante génétique.

Par origine externe, on entend l'incidence de la flore locale du cuir chevelu, l'action d'agents chimiques (colorations, permanentes, chimiothérapies), la pollution ou l'hygiène de vie.

Tous ces désordres d'origine interne ou externe ciblant le cuir chevelu ont pour conséquence directe la perturbation et la modification du cycle pilaire conduisant à différentes formes d'alopécies. Le terme d'alopécie résume l'ensemble des pathologies atteignant le follicule pileux et le cycle de croissance de la tige pilaire et ayant pour conséquence la perte occasionnelle et réversible ou la chute définitive de tout ou partie des cheveux. On citera les deux types d'alopécies les plus répandus, l'alopécie aerata et l'alopécie androgénique ou chronique.

Par "extrait de *Cleome spinosa*", on entend au sens de la présente invention un extrait de *Cleome spinosa* contenant de préférence une teneur totale en soufre comprise entre 0,05g et 5g pour 100g de matière sèche de l'extrait.

L'extrait de *Cleome spinosa* se caractérise chimiquement par sa teneur en soufre qui prend la forme d'acides aminés soufrés comme la cystine, la méthionine. Le soufre total dosé après minéralisation et dosage par émission plasma présente des teneurs variant selon les solvants et les conditions d'extraction de 0,05 g à 5g pour 100 g de matière sèche de l'extrait. La cystine peut être dosée par Chromatographie Liquide Haute Performance, ses teneurs varient également selon les conditions d'extraction de 0,01 g à 1g pour 100 g de matière sèche de l'extrait. Les teneurs en méthionine sont plus faibles, elles varient selon les conditions d'extraction de 0,001 g à 0,1 g pour 100 g de matière sèche.

De façon avantageuse, l'extrait de *Cleome spinosa* selon la présente invention est obtenu à partir des feuilles, fleurs, fruits, tiges, racines de *Cleome spinosa ;* et préférentiellement des parties aériennes fleuries sèches.

La présente invention concerne l'utilisation d'un tel extrait en tant que principe actif pour la préparation d'une composition pharmaceutique destinée à prolonger la croissance des follicules pileux.

Préférentiellement, la composition pharmaceutique ou cosmétique selon la présente invention comprend une quantité d'extrait sec de *Cleome spinosa,* à titre de principe actif, comprise entre 5 mg et 1 g pour 100 g de ladite composition.

Avantageusement, ladite quantité d'extrait sec de *Cleome spinosa* est comprise entre 10 mg et 500 mg pour 100 g de composition pharmaceutique ou cosmétique. De façon encore plus avantageuse, ladite quantité d'extrait sec de *Cleome spinosa* est comprise entre 20 mg et 100 mg pour 100 g de composition.

Au sens de la présente invention, l'extrait de *Cleome spinosa* agit comme principe actif pour stimuler et prolonger la croissance de follicules pileux.

L'extrait selon la présente invention favorise le développement de la tige pilaire, améliore l'apport des éléments qui rentrent dans la constitution du cheveu, favorise le maintien du cheveu très proche du tissu sous cutané qui caractérise la phase anagène.

La phase de croissance active (phase anagène) s'en trouve améliorée, prolongée.

La composition selon la présente invention peut contenir en outre d'autres actifs bien connus de l'homme du métier pour le traitement anti-chute des cheveux: on citera de façon non limitative : un extrait de quinquina (FR 2 853 245), le minoxidil ou 2,4-diamino-6-pipéridinopyrimidine-3-oxide, des vitamines telles que les vitamines A, E, B5, B6, C, H, PP, des oligo-éléments tels que le zinc, le cuivre, le magnésium, le silicium, etc, des dérivés protéiques tels que les peptides, les acides aminés soufrés (type méthionine, cystine, cystéine ou dérivés), des huiles essentielles ou des extraits d'origine végétale de nature lipophile ou hydrophile dont la liste n'est pas limitative, des agents antifongiques tels que la piroctonolamine, les dérivés undécyléniques, la ciclopiroxolamine, ou des molécules de synthèse chimique réputées pour leur action spécifique au niveau des récepteurs androgéniques ou sur la synthèse ou l'expression des 5-$\alpha$-réductases.

Enfin, ladite préparation peut aussi contenir des excipients pharmaceutiquement acceptables adaptés pour l'administration par voie orale ou par voie topique.

Ladite composition se présente de préférence sous forme topique ou orale ; et encore plus préférentiellement sous forme topique.

Avantageusement, la forme topique est choisie parmi le groupe constitué d'un shampoing, gel, lotion, mousse, spray, crème. Cette composition peut aussi se présenter sous une forme orale, telle qu'une capsule, comprimé, gélule, poudre pour suspensions buvables.

Dans un mode de réalisation particulier de l'invention, la composition selon la présente invention peut être utilisée en tant que complément alimentaire ou composition diététique pour prolonger la croissance de follicules pileux.

Ledit extrait de *Cleome spinosa* selon la présente invention peut être obtenu comme suit :

- broyage des parties aériennes fleuries séchées (feuilles et/ou de fleurs et/ou de fruits et/ou de tiges) de *Cleome spinosa*
- au moins une extraction par un solvant qui peut être l'eau, un alcool de $C_1$ à $C_4$, une cétone (méthyl éthylcétone, diméthylcétone, méthylisobutylcétone), un ester (acétate d'éthyle, acétate d'isopropyle) et un mélange en toute proportion miscible de ces solvants.

L'extraction est réalisée dans un ratio plante/solvant compris entre environ 1/1 et 1/20 et peut être renouvelée 2 à 3 fois. La température d'extraction peut être égale à la température ambiante jusqu'à la température d'ébullition du solvant d'extraction.

La durée de l'extraction peut varier de 30 minutes à 72 heures et peut se faire de façon statique comme sous agitation : séparation solide/liquide par des techniques connues de l'homme du métier. Il peut s'agir d'une opération de filtration ou de centrifugation par exemple.

La solution obtenue peut être utilisée comme telle si le solvant d'utilisation est compatible avec son utilisation. Elle peut également être plus ou moins concentrée jusqu'à l'obtention d'un extrait sec. La solution concentrée peut être également utilisée.

Un traitement de décoloration peut être entrepris en rajoutant à la solution extraite concentrée ou non un support absorbant comme le charbon actif ou une résine absorbante. Lors de l'étape de séchage, il peut être rajouté un support dans les proportions massiques par rapport à la matière première extraite pouvant varier de 1 à 50 %. Le support peut être une maltodextrine, un sucre comme le lactose ou de la silice. Lors de l'étape de concentration, on peut également rajouter un solvant à haut point d'ébullition comme la glycérine, le propylène glycol, le butylène glycol, l'hexylène glycol. L'extrait de *Cleome spinosa* est alors solubilisé dans ce nouveau solvant.

Les préparations et les compositions suivantes sont citées à titre d'exemples illustratifs et non limitatifs.

EXEMPLES DE PREPARATION DE L'EXTRAIT VEGETAL

Exemple 1

100 kg de parties aériennes fleuries séchées et broyées de *Cleome spinosa* sont extraites par 1500 kg d'alcool 95 % (v/v) sous agitation à reflux pendant 1 heure. Après refroidissement la solution extraite est récupérée par filtration.

A cette solution on rajoute 15 kg de charbon actif et on porte à reflux pendant 20 minutes. Le charbon est retiré par filtration. On obtient ainsi une solution limpide jaune clair. Cette solution est concentrée puis séchée. On obtient 10 kg d'extrait sec. Sa teneur en soufre total est de 0,5 g pour 100 g de matière sèche, en cystine de 0,1 g pour 100 g de matière sèche et en méthionine de 0,015 g pour 100 g de matière sèche.

Exemple 2

10 kg de parties aériennes fleuries séchées et broyées de *Cleome spinosa* sont extraits avec 70 kg d'alcool 30 % (v/v) sous reflux pendant 2 heures et sous agitation. Une fois la solution recueillie après filtration, la drogue est de nouveau extraite dans les mêmes conditions que précédemment par 70 kg d'alcool 30 % (v/v). Les solutions obtenues sont jointes. On obtient ainsi 120 kg d'un extrait liquide jaune foncé. La teneur en soufre total est 1 g pour 100 g de matière sèche de l'extrait liquide, en cystine de 0,2 g pour 100 g et de 0,02 g pour 100 g en méthionine.

Exemple 3

1 kg de parties aériennes fleuries séchées et broyées de *Cleome spinosa* est extrait par 10 litres d'eau à 80° C pendant 5 heures et sous agitation. La solution obtenue par filtration est concentrée jusqu'à 2 litres, on ajoute alors 2 l de propylène glycol et on filtre. On obtient ainsi 4 litres d'un extrait liquide marron clair. Pour 100 g de matière sèche de cet extrait, la quantité de soufre total est de 1 g, en cystine de 0,2 g et en méthionine de 0,02 g.

EXEMPLE DE COMPOSITION COSMETIQUE

Exemple 4 : lotion antichute

| Composé | Quantité (/ 100g) |
|---|---|
| Extrait sec *Cleome spinosa* | 20 mg à 100 mg |
| Extrait quinquina | 3 g à 6 g |
| Alcool éthylique 96% | qs |
| Hexylene glycol | 2,5 % |
| Vitamine PP | 1 % |
| Vitamine H | 0,003 % |
| Acide Béta glycyrrhetinique | 0,3 % |
| Décaméthylcyclopentasiloxane | qs |
| Eau purifiée | Qsp 100 g |

EVALUATION PHARMACOLOGIQUE

Etude de l'effet d'un extrait de *Cleome spinosa* sur la croissance de follicules pileux humains en culture.

L'exploration de l'impact de différents facteurs sur la croissance pilaire peut être étudiée aussi bien *in vivo* qu'*in vitro.* L'établissement de modèles de culture simplifiés représentatifs de tous les aspects du comportement du follicule pileux *in vivo* est une étape indispensable à l'optimisation des évaluations des produits antichute.

La culture de follicule pileux isolé a été développée par Philpott *and al.* en 1990. Il a montré une croissance des follicules durant 10 jours.

Plusieurs équipes ont ensuite utilisé des variantes de ce modèle par l'addition de facteurs de croissance ou d'autres entités moléculaires impliquées dans les mécanismes de contrôle de la croissance pilaire : Buhl Al and al. J Invest Dermatol, 1992, Mars, 98 (3) : 315-319 ; Harmon CS and al., Lymphokine Cytokine Res., 1993, Aug 12 (4) : 197-203 ; Philpott MP and al., J Dermatol Sci. 1994 Jul, 7 suppl : 573-78.

L'extrait de *Cleome spinosa* obtenu selon l'exemple 1 a été évalué sur la croissance directe de follicules pileux entiers de scalp humain *ex vivo* durant 14 jours de culture. Le minoxidil, molécule de référence, a été menée en parallèle.

Les follicules pileux sont issus de scalp humain provenant du service de chirurgie plastique des hôpitaux ou des cliniques (deux donneurs).

La peau est rasée puis lavée dans 6 bains d'antibiotiques. Les follicules en phase anagène reconnaissables car très vascularisés, sont ensuite isolés de la peau sous la loupe binoculaire et débarrassés de la graisse.

Il sont ensuite incubés à 37°C dans des boîtes de culture 24 puits dans un milieu de culture adéquat contenant les produits à tester.

La croissance de la tige des follicules pileux est mesurée à l'aide de papier millimétré sous loupe binoculaire aux jours 0, 4, 5, 7, 9, 12 ou 14.

La détermination des pourcentages de croissance et d'activité de l'extrait hydroalcoolique de *Cleome spinosa* a été réalisée après 4 ou 5, 7 ou 8, 12 et 14 jours de traitement des follicules pileux en comparaison avec des follicules témoins non traités.

On note :

Tx le temps x après traitement

$T_0$ le temps 0 début de traitement

```
     Le pourcentage de croissance est égal à (mesure T_x - mesure
T_t)/mesure T_0 x 100
```

L'activité du traitement est calculée de la façon suivante:

```
              [(mesure traité T_x - mesure traité T_0) - (mesure
contrôle T_x - mesure contrôle T_0)]/(mesure contrôle T_x - mesure
contrôle T_0) x 100
```

Les résultats sont résumés dans le tableau suivant :

|  | % activité | | | |
| --- | --- | --- | --- | --- |
|  | J4/J5 | J8/J7 | J12 | J14 |
| Minoxidil | 55 | 41 | 21 | 17 |
| Extrait de *Cleome spinosa* | | | | |
| 10 $\mu$g/ml | 40 | 25 | 38 | 48 |
| 1 $\mu$g/ml | 27 | 34 | 22 | 23 |
| 0,1 $\mu$g/ml | 65 | 31 | 31 | 32 |

On observe une stimulation de la croissance des follicules pileux humains en culture pour toutes les concentrations testées de l'extrait de *Cleome spinosa.*

**Revendications**

1.   Extrait de *Cleome spinosa* en tant que principe actif d'une composition pharmaceutique pour son utilisation pour le traitement des désordres capillaires.

2.   Extrait pour son utilisation selon la revendication 1, **caractérisée en ce qu'**il contient de 0,01 g à 1g de cystine pour 100 g de matière sèche.

3.   Extrait pour son utilisation selon l'une des revendications 1 et 2, **caractérisée en ce qu'**il contient de 0,001 g à 0,1 g de méthionine pour 100 g de matière sèche.

4.   Extrait pour son utilisation selon l'une des revendications 1 à 3, **caractérisée en ce qu'**il est présent dans la composition à raison d'une quantité comprise entre 5 mg et 1g pour 100 g de ladite composition.

5.   Extrait pour son utilisation selon l'une des revendications 1 à 3, **caractérisée en ce qu'**il est présent dans la composition à raison d'une quantité comprise entre 10 mg et 500 mg pour 100 g de ladite composition.

6.   Extrait pour son utilisation selon l'une des revendications 1 à 3, **caractérisée en ce qu'**il est présent dans la composition à raison d'une quantité comprise entre 20 mg et 100 mg pour 100 g de ladite composition.

7.   Extrait pour son utilisation selon l'une des revendications 1 à 6, **caractérisé en ce qu'**il est présent dans la composition aux côtés d'un ou plusieurs autre(s) principe(s) actif(s) choisi(s) parmi un extrait de quinquina, le minoxidil ou 2,4-diamino-6-pipéridinopyrimidine-3-oxide, des vitamines telles que les vitamines A, E, B5, B6, C, H, PP, des oligo-éléments tels que le zinc, le cuivre, le magnésium, le silicium, des dérivés protéiques tels que les peptides, les acides aminés soufrés du type méthionine, cystine, cystéine ou dérivés, des huiles essentielles ou des extraits d'origine végétale de nature lipophile ou hydrophile, des agents antifongiques tels que la piroctonolamine, les dérivés undécyléniques, la ciclopiroxolamine.

8. Extrait pour son utilisation selon la revendication 7, **caractérisé en ce qu'**il est présent dans la composition en combinaison avec un extrait de quinquina.

9. Procédé cosmétique pour prolonger la croissance de follicules pileux, **caractérisé en ce qu'**il implique l'utilisation par voie topique ou orale d'un extrait de *Cleome spinosa.*

## Patentansprüche

1. *Cleome spinosa*-Extrakt als Wirkstoff einer pharmazeutischen Zusammensetzung zur Verwendung zur Behandlung von Haarstörungen.

2. Extrakt zur Verwendung nach Anspruch 1, **dadurch gekennzeichnet, dass** er 0,01 g bis 1 g Cystin pro 100 g Trockensubstanz enthält.

3. Extrakt zur Verwendung nach einem der Ansprüche 1 und 2, **dadurch gekennzeichnet, dass** er 0,001 g bis 0,1 g Methionin pro 100 g Trockensubstanz enthält.

4. Extrakt zur Verwendung nach einem der Ansprüche 1 bis 3, **dadurch gekennzeichnet, dass** er in der Zusammensetzung in einer Menge zwischen 5 mg und 1 g pro 100 g der Zusammensetzung vorliegt.

5. Extrakt zur Verwendung nach einem der Ansprüche 1 bis 3, **dadurch gekennzeichnet, dass** er in der Zusammensetzung in einer Menge zwischen 10 mg und 500 mg pro 100 g der Zusammensetzung vorliegt.

6. Extrakt zur Verwendung nach einem der Ansprüche 1 bis 3, **dadurch gekennzeichnet, dass** er in der Zusammensetzung in einer Menge zwischen 20 mg und 100 mg pro 100 g der Zusammensetzung vorliegt.

7. Extrakt zur Verwendung nach einem der Ansprüche 1 bis 6, **dadurch gekennzeichnet, dass** er in der Zusammensetzung neben einem oder mehreren anderen Wirkstoff(en), ausgewählt aus einem Chinarinden-Extrakt, Minoxidil oder 2,4-Diamino-6-piperidinpyrimidin-3-oxid, Vitaminen wie Vitamin A, E, B5, B6, C, H, PP, Spurenelementen wie Zink, Kupfer, Magnesium, Silicium, Proteinderivaten wie Peptiden, schwefelhaltigen Aminosäuren vom Typ Methionin, Cystin, Cystein oder Derivaten, essentiellen Ölen oder lipophilen oder hydrophilen Extrakten pflanzlichen Ursprungs, Antimykotika wie Piroctonolamin, Undecylenderivaten, Ciclopiroxolamin, vorliegt.

8. Extrakt zur Verwendung nach Anspruch 7, **dadurch gekennzeichnet, dass** er in der Zusammensetzung in Kombination mit einem Chinarinden-Extrakt vorliegt.

9. Kosmetisches Verfahren zur Verlängerung des Wachstums von Haarfollikeln, **dadurch gekennzeichnet, dass** es die Verwendung eines *Cleome spinosa*-Extrakts auf topischem oder oralem Weg beinhaltet.

## Claims

1. *Cleome spinosa* extract as an active ingredient of a pharmaceutical composition for its use for treating hair disorders.

2. Extract for its use according to claim 1, **characterised in that** it contains from 0.01 g to 1 g of cystine per 100 g of dry matter.

3. Extract for its use according to one of claims 1 and 2, **characterised in that** it contains from 0.001 g to 0.1 g of methionine per 100 g of dry matter.

4. Extract for its use according to one of claims 1 to 3, **characterised in that** it is present in the composition in an amount ranging between 5 mg and 1 g per 100 g of said composition.

5. Extract for its use according to one of claims 1 to 3, **characterised in that** it is present in the composition in an amount ranging between 10 mg and 500 mg per 100 g of said composition.

6. Extract for its use according to one of claims 1 to 3 **characterised in that** it is present in an amount ranging between 20 mg and 100 mg per 100 g of said composition.

7.  Extract for its use according to one of claims 1 to 6, **characterised in that** it is present in the composition alongside one or more other active ingredients selected from a quinquina extract, minoxidil or 2,4-diamino-6-piperidinopyrimidine-3-oxide, vitamins such as vitamins A, E, B5, B6, C, H, PP, trace elements such as zinc, copper, magnesium, silicon, protein derivatives such as peptides, sulphur-containing amino acids of the methionine, cystine, cysteine type or derivatives, essential oils or extracts of plant origin with lipophilicity or hydrophilicity, antifungal agents such as piroctonolamine, undecylenic derivatives, ciclopirox olamine.

8.  Extract for its use according to claim 7, **characterised in that** it is present in the composition in combination with a quinquina extract.

9.  Cosmetic method for extending the growth of hair follicles **characterised in that** it involves the use via a topical or oral route of a *Cleome spinosa* extract.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 2001181129 B

- FR 2853245

**Littérature non-brevet citée dans la description**

- **DWIGHT.** *J. Nat. Prod.,* 2004, vol. 67, 179-183
- **PAGANI K.** Il Farmaco. 1967, vol. 22, 553
- **BUHL AL.** *J Invest Dermatol,* Mars 1992, vol. 98 (3), 315-319

- **HARMON CS.** *Lymphokine Cytokine Res.,* 12 Août 1993, 197-203
- **PHILPOTT MP.** *J Dermatol Sci.,* Juillet 1994, vol. 7, 573-78